# FASCICULE DE BREVET EUROPEEN

(11) **EP 0 918 759 B1**
(45) Date de publication et mention de la délivrance du brevet: **28.08.2002**
(21) Numéro de dépôt: 97925034.7
(22) Date de dépôt: 02.06.1997
(51) Int. Cl.: C07D 277/34

(54) **NOUVEAUX DERIVES DE THIAZOLIDINE-2,4-DIONE SUBSTITUES, LEURS PROCEDES D'OBTENTION ET LES COMPOSITIONS PHARMACEUTIQUES EN RENFERMANT**
SUBSTITUIERTE THIAZOLIDINEDIONE DERIVATE, IHRE HERSTELLUNG UND PHARMAZEUTISCHE ZUSAMMENSETZUNGEN, DIE SIE ENTHALTEN
NOVEL THIAZOLIDONE-2 DERIVATIVES, 4-DIKETONE SUBSTITUTED, METHOD FOR OBTAINING THEM AND PHARMACEUTICAL COMPOSITIONS CONTAINING SAME

(30) Priorité: 07.06.1996 FR 9607070
(43) Date de publication de la demande: 02.06.1999
(73) Titulaire: MERCK PATENT GmbH, 64293 Darmstadt (DE)
(72) Inventeur: MOINET, Gérard, F-91400 Orsay (FR); BOTTON, Gérard, F-78530 Buc (FR); PRUGNARD, Etienne, F-75013 Paris (FR); DOARE, Liliane, F-91170 Viry Châtillon (FR); KERGOAT, Micheline, F-91440 Bures sur Yvette (FR); MESANGEAU, Didier, F-77380 Combs la Ville (FR)
(86) Numéro de dépôt international: EP9702851
(87) Numéro de publication internationale: WO97047612

(56) Documents cités:
- EP-A- 0 008 203
- EP-A- 0 612 743
- EP-A- 0 643 050
- WO-A-96/05186
- CHEMICAL AND PHARMACEUTICAL BULLETIN, vol. 30, no. 10, Octobre 1982, TOKYO JP, pages 3580-3600, XP002024759 TAKASHI SOHDA ET AL: "Studies on antidiabetic agents." cité dans la demande

## Description

La présente invention se rapporte au domaine de la chimie et plus particulièrement à celui de la chimie thérapeutique.

La présente invention a plus précisément pour objet de nouveaux dérivés de la thiazolidine-2,4-dione, à savoir les 5-phénoxyalcoyl-2,4-thiazolidinediones, leurs procédés d'obtention, leur utilisation en tant qu'agents antidiabétiques et dans le traitement du syndrome métabolique de l'insulinorésistance, et les compositions pharmaceutiques qui les contiennent.

De nombreux dérivés de la thiazolidine-2,4-dione ont déjà décrits en tant qu'agents antidiabétiques (Takeda, brevet EP 193 256, Sankyo brevet EP 207 581).
Les composés décrits précédemment étaient principalement des thiazolidines diones substituées en position 5 par un radical benzyle, c'est-à-dire des composés ayant une chaîne alcoylène ne comportant qu'un seul atome de carbone entre le noyau thiazolidine et un groupement aryle.
La structure de ces composés comportait en général des variations sur le substituant porté par le cycle aryle du radical benzyle.
Des composés possédant les structures antérieurement décrites et présentant des activités hypoglycémiantes et hypotriglycéridemiantes notables, avaient pour enchaînement en position 5 le groupement R-O-Ar-CH₂-.
Ces variations portaient exclusivement sur le substituant R porté par l'oxygène en position para du phényle.

Certains de ces composés à côté de leurs propriétés pharmacologiques manifestent des phénomènes d'hépatotoxicité. (Takashi Sohda, Chem. Pharm. Bull 30 (1982) 3580).
On sait que dans le diabète non-insulinodépendant, une diminution de l'efficacité de l'insuline conduit à l'hyperglycémie.
La diminution de « l'activité » de l'insuline est liée, d'une part, à un défaut pancréatique de réponse insulinique au glucose et, d'autre part, à une insulinorésistance hépatique et périphérique (muscles - tissus adipeux).

Certaines thérapeutiques antidiabétiques actuelles stimulent principalement la sécrétion d'insuline, sans améliorer l'insulinorésistance, et ont pour défaut majeur, à long terme, d'aggraver le diabète par épuisement des cellules β pancréatiques.
D'autres antihyperglycémiants améliorent la sensibilité à l'insuline tels que la Metformine et les composés possédant la structure thiazolidine-2,4-dione.
Ces thiazolidinediones abaissent la glycémie sans stimuler la sécrétion d'insuline et s'avèrent plus actives dans l'insulinorésistance avec hyperinsulinisme.

Les composés de la présente invention sont nouveaux et, se différencient des autres dérivés de la thiazolidine-2,4-dione par des propriétés que les composés de la littérature antérieure ne possédaient pas : absence d'effet sur la sécrétion d'insuline, action sur l'insulinorésistance, absence d'effet hépatonocif, activité chez les diabétiques dans le cas de diabète sans hyperinsulinisme.

La présente invention a spécifiquement pour objet les nouvelles 5-phénoxyalcoyl-2,4-thiazolidinediones répondant à la formule générale I : 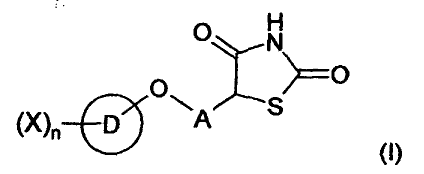 dans laquelle A représente CH₂CH₂, éventuellement substituée par un radical hydroxyle ou par un radical phényle,
D représente une structure aromatique mono-, bi- ou tricyclique, homo- ou hétérocarbonée pouvant inclure un ou plusieurs hétéroatomes
X représente un substituant de la structure aromatique, choisi parmi l'hydrogène, un groupe alcoyle ayant de 1 à 6 atomes de carbone, un groupe alcoxy ayant de 1 à 6 atomes de carbone, un groupe alcoxyalcoyle dans lequel les groupements alcoxy et alcoyle sont définis comme précédemment, un groupe aryle défini comme une structure cyclique aromatique comportant un ou deux cycles incluant éventuellement un ou deux hétéroatomes dans le cycle comme par exemple un phényle ou un α- ou un β-naphtyle, un groupe aralcoyle dans lequel le groupement alcoyle est défini comme précédemment et le groupement aryle est défini comme précédemment, un groupe aralcoylaryle dont les fractions aralcoyle et aryle sont définis comme précédemment, un halogène, un trifluorométhyle, un cyano, un hydroxy, un nitro, un amino, un carboxyle, un alcoxy carbonyle, un
carboxamide, un sulfonyle, un sulfone, un sulfonamide, un sulfamoyle, un alcoylsulfonylamino, un acylamino, un trifluorométhoxy n est un nombre entier allant de 1 à 3.

Dans ce qui précède, parmi les radicaux aromatiques D, on pourra citer comme structure homocarbonée le radical phényle, α-naphtyle, β-naphtyle ou fluorényle.
Parmi les radicaux aromatiques hétérocycliques, on pourra citer le pyridyle, le cycle quinoléinyle ou carbazolyle.
Pour autant que l'invention est concernée, un groupe alcoyle est défini comme ayant de 1 à 6 atomes de carbone et notamment un radical méthyle, éthyle, propyle, isopropyle, butyle, isobutyle, tertiobutyle, pentyle...., un groupe alcoxy est défini comme ayant de 1 à 6 atomes de carbone et notamment un radical méthoxy, éthoxy, propoxy, isopropoxy, butoxy, isobutoxy..., un groupe halogène est défini comme étant le fluor, le chlore, le brome, l'iode.
La chaîne alcoylène A est une chaîne hydrocarbonée ayant de 2 atomes de carbone, linéaire, éventuellement substituée par un radical hydroxyle ou par un radical phényle. Un exemple d'une telle chaîne sera un radical éthylène.

La présente invention se rapporte également aux formes tautomères des composés de formule générale I, aux énantiomères, diastéréoisomères et épimères de ces composés, ainsi qu'à leurs solvates.

On peut concevoir que les fonctions cétones portées par le cycle thiazolidine peuvent s'énoliser et donner naissance à des mono-énols.
Les dérivés de thiazolidinedione de formule générale I qui possèdent un proton acide sur l'azote du cycle thiazolidinedione peuvent dans ce cas être salifiés et se présenter sous forme de sels basiques.
Des exemples de sels basiques des composés de formule générale I incluent les sels pharmacologiquement-acceptables, tels que les sels de sodium, sels de potassium, sels de magnésium, sels de calcium, sels d'amine et autres sels du même type (aluminium, fer, bismuth, etc..). Les sels d'amine qui ne sont pas pharmacologiquement-acceptables peuvent servir de moyen d'identification, de purification ou de dédoublemnt.

Parmi les composés de formule générale I selon l'invention, on citera plus particulièrement, à titre de composés actuellement préférés :
- la 5-(2-phénoxyéthyl) thiazolidine-2,4-dione
- la 5-[1-méthyl-2-phénoxyéthyl] thiazolidine-2,4-dione
- la 5-[2-(4-cyanophénoxy)ethyl] thiazolidine-2,4-dione
- la 5-[2-(2-naphtyloxy)ethyl] thiazolidine-2,4-dione
et leurs sels pharmacologiquement acceptables.

L'invention concerne également les procédés pour obtenir les 5-phénoxyalcoyl-2,4-thiazolidinedione de formule générale I.

Un procédé de synthèse selon l'invention, (voie A), est une synthèse malonique, qui consiste en ce que l'on soumet un composé de formule II : 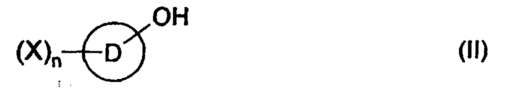 dans lequel X, D et n sont définis comme précédemment, à l'action d'un dihalogénoalcoyle de formule III : 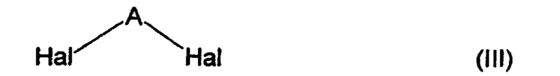 dans laquelle Hal représente un atome de chlore ou de brome,
A est un radical alcoylène tel que défini précédemment,
en présence d'un agent basique,
pour former un composé de formule générale IV : 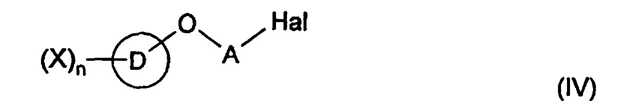 dans laquelle X, D, n et A sont définis tels que précédemment,
que l'on soumet à l'action d'un malonate de dialcoyle, de formule V : 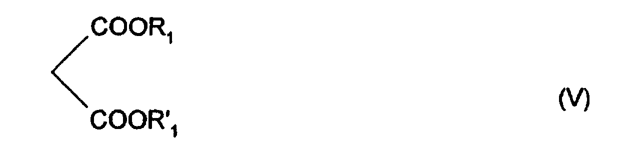 dans laquelle R₁ et R'₁ sont des radicaux alcoyle,
en présence d'un alcoolate de métal alcalin,
pour former un composé de formule générale VI 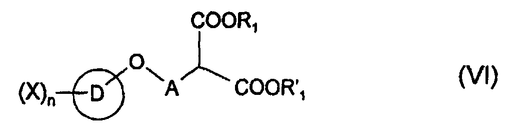 dans laquelle X, D, n, A, R₁ et R'₁ sont définis comme précédemment,
qui est soumis à une halogénation par action d'un agent halogénant pour former le composé de formule générale VII : 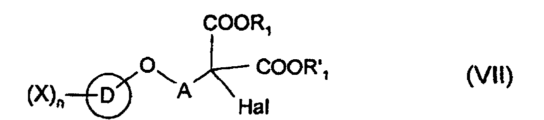 dans laquelle Hal représente un atome de chlore ou de brome,
X, D, n, A, R₁ et R'₁ sont définis comme précédemment.
Le composé malonique VI peut être halogéné par un N - halogénoamide ou un N-halogénoimide après formation de l'anion, comme par exemple par action de l'hydrure de sodium dans le tétrahydrofuranne.
Le diester dialcoylique de formule générale VII est décarboxylé et saponifié par chauffage dans un mélange acide formé notamment d'acide chlorhydrique et d'acide acétique pour obtenir l'α-halogénoacide de formule générale VIII : 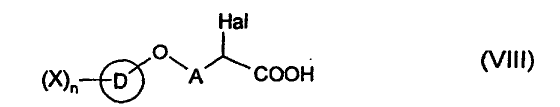 dans laquelle Hal, X, D, n et A sont définis comme précédemment,
qui est mis en réaction avec la thiourée pour former une 2-imino-4-thiazolidinone de formule générale IX : 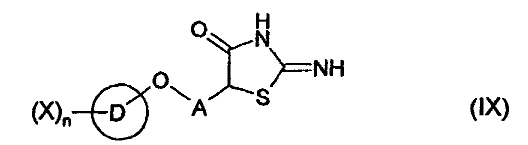 dans laquelle les groupements X, D, n et A sont définis comme précédemment,
qui, sans être nécessairement isolée, est hydrolysée en thiazolidine-2,4-dione de formule générale I, en ajoutant un mélange acide comme de l'acide chlorhydrique.
Cette hydrolyse s'effectue de préférence en chauffant à reflux.
Dans ce procédé, l'agent basique utilisé pour former le composé de formule générale IV est de préférence un hydroxyde de métal alcalin et notamment la soude.

De même, l'halogénoamide pourra être la N-chloroacétamide, le N-bromoacétamide ou la N-bromobenzamide et l'halogénoimide pourra être la N-chlorosuccinimide ou la N-chlorophtalimide.

Un autre procédé de synthèse par la voie malonique (voie B) consiste à soumettre un composé de formule X : 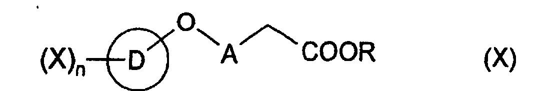 dans laquelle R représente un groupe alcoyle,
X, D, n et A sont tels que définis précédemment,
à une halogénation pour former l'ester α-halogéné de formule générale XI : 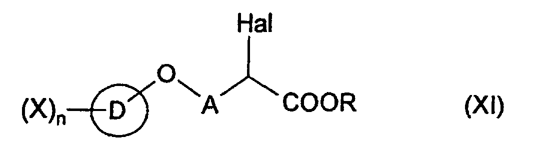 dans laquelle Hal représente un atome de chlore ou de brome,
X, D, n et A sont tels que définis précédemment,
puis à mettre en réaction ce dernier avec la thiourée en présence d'un agent tampon comme l'acétate de sodium pour former la 2-imino-4-thiazolidinone de formule IX : 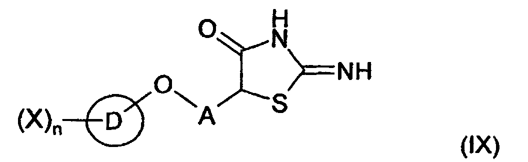 dans laquelle X, D, n et A sont tels que définis précédemment,
qui est hydrolysée par chauffage à reflux dans l'acide chlorhydrique, pour former la thiazolidinedione de formule générale I : 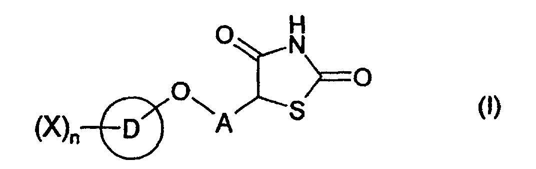 dans laquelle D représente une structure aromatique mono-, bi- ou tricyclique, homo-ou hétérocarbonée pouvant inclure un ou plusieurs hétéroatomes,
X représente un substituant de la structure aromatique et est défini comme précédemment,
A représente CH₂CH₂.

Un autre procédé de synthèse, selon l'invention (voie C), consiste en ce que l'on soumet un composé halogéné de formule IV : 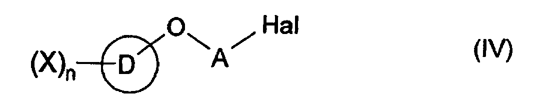 dans laquelle Hal représente un atome de chlore ou de brome,
X, D, n et A sont définis comme précédemment,
à l'action du dianion de la thiazolidine-2,4-dione obtenu par action d'un dérivé alcalin comme le butyllithium sur la thiazolidine-2,4-dione pour former un composé de formule générale I.

Dans un autre procédé selon l'invention (voie D), la synthèse part d'un aldéhyde aryloxyalcoylique et consiste en ce que l'on soumet le composé aldéhydique de formule XII: 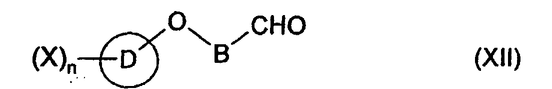 dans laquelle B représente CH₂,
X, D et n sont tels que définis précédemment,
à l'action du dianon de la thiazolidine-2,4-dione obtenu par action d'un dérivé alcalin sur la thiazolidine-2,4-dione,
pour former un composé de formule générale XIII : 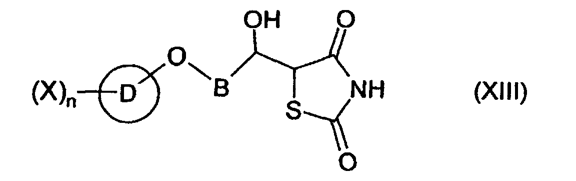 dans lequel X, D et n sont tels que définis précédemment, et B représente CH₂.

Ce composé est ensuite transformé en composé déshydroxylé de formule I, par déshydratation puis hydrogénation sélective, ou bien encore par réduction de la fonction alcool en dérivé saturé.

Un autre procédé d'obtention des composés de formule générale I (voie F), consiste au départ d'une cétone à soumettre ladite cétone de formule générale XVI : 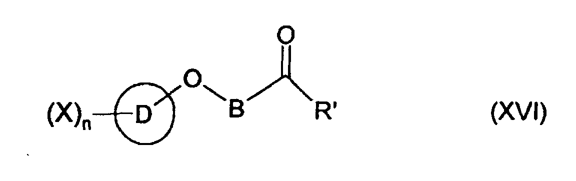 dans laquelle R' représente un groupe alcoyle linéaire ou ramifié, ou un groupe aryle ou aralcoyle, substitués ou non,
X, D, n sont définis comme précédemment, et B représente CH₂,
à l'action de la thiazolidine-2,4-dione en présence d'une base organique pour former, après déshydratation du carbinol intermédiaire en milieu acide, le composé de formule générale XVII : 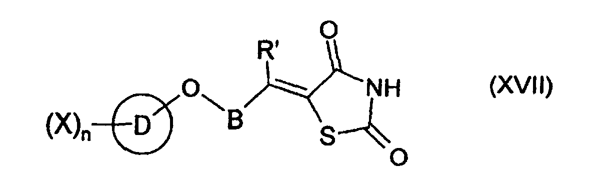 dans laquelle X, D, n et R' sont définis comme précédemment, et B représente CH₂, puis à réduire la double liaison par hydrogénation en présence d'un catalyseur, pour former le composé de formule générale I, dans laquelle la chaîne alcoylène est une chaîne ramifiée : 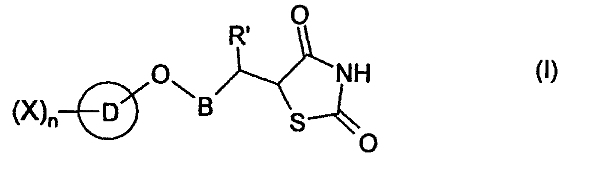 X, D, n et R' sont définis comme précédemment, et B représente CH₂.

L'hydrogénation catalytique du composé XVII s'effectue de préférence en présence d'un métal de la famille du platine sur un support inerte comme par exemple le charbon palladié, le charbon platiné ou le palladium sur carbonate de calcium.

A titre de récapitulation, dans les procédés de synthèse C, D, E et F, les composés de formule générale I sont obtenus par action de différents agents électrophiles tels que décrits ci-après, de manière non limitative sur le dianion de la thiazolidine-2,4-dione, de préférence à basse température.

Ce dianion peut être obtenu par action d'une base forte telle que le diéthylamidure de lithium, l'amidure de lithium, le diisopropylamidure de lithium, le n-BuLi, sur la thiazolidine-2,4dione.

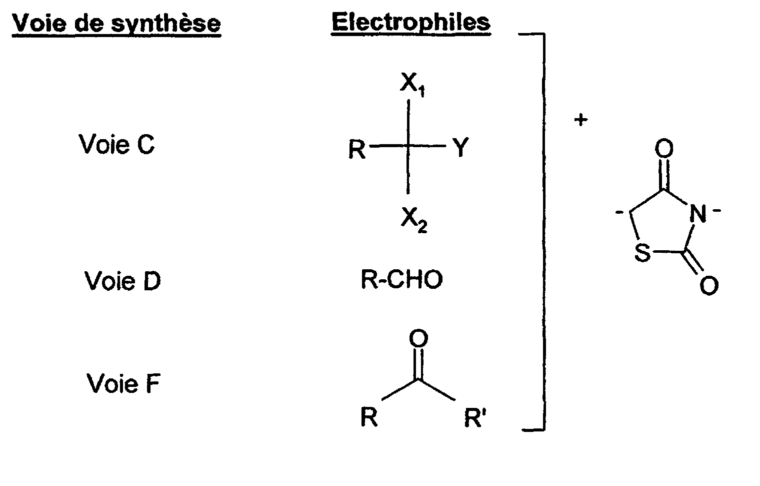

**R représente :** 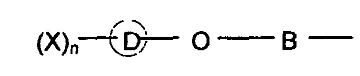 ou 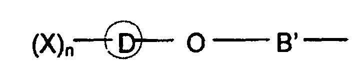
D est défini comme précédemment,
X représente le substituant de la structure aromatique et est défini comme précédemment,
B représente CH₂CH₂,
B' représente CH₂,
Y représente un atome de brome ou de chlore ou un radical méthylsulfonyloxy ou p.toluène sulfonyloxy,
R' représente un groupement alcoyle, linéaire ou ramifié,
ou bien R' représente un groupement aryle ou aralcoyle, substitués ou non,
X₁ représente un hydrogène ou un groupement alcoyle, linéaire ou ramifié,
ou bien X₁ représente un groupement aryle ou aralcoyle, substitués ou non,
X₂ représente un hydrogène ou un groupement alcoyle, linéaire ou ramifié,
ou bien X₂ représente un groupement aryle ou aralcoyle, substitués ou non.

Les composés selon l'invention manifestent des propriétés pharmacologiques très intéressantes et trouvent, de ce fait, un emploi en thérapeutique.

Les composés selon l'invention se différencient des autres dérivés de la thiazolidine-2,4-dione par l'intensité de leur activité antidiabétique dans des modèles de diabète sans hyperinsulinisme où les composés de l'art antérieur, comme par exemple la troglitazone, s'avèrent peu actifs.

Ainsi les composés de l'invention sont utilisables dans le traitement des états diabétiques non insulinopéniques, permettant d'obtenir un meilleur contrôle de la glycémie en ayant un taux d'insuline circulant, diminué.

La prévention de cet hyperinsulinisme relatif, associée à une amélioration des dyslipidémies et à une activité antioxydante, peut concourir à une réduction des risques micro et macroangiopathiques.

Les composés de l'invention sont utilisables dans le traitement du syndrome métabolique de l'insulinorésistance, impliquant un effet thérapeutique bénéfique sur le diabète non-insulinodépendant, l'hyperinsulinisme, l'hypertension et les dyslipidémies mais également dans les diabètes non-insulinodépendants avec hypoinsulinisme.

Les composés trouvent, de plus, une utilisation dans le traitement de l'hypertension des sujets insulinorésistants, associés ou non à d'autres anomalies métaboliques.

L'activité diurétique et la diminution de la capture du Ca²⁺ observée sur l'aorte de rat peuvent engendrer une activité antihypertensive pour certains des composés de formule générale I.

Certains des composés possèdent, en outre, des activités antiradicalaires vis-à-vis de l'anion hydroxyle et superoxide, mises en évidence à l'aide d'un modèle d'investigation dit cellulaire.

A ces fins, les composés selon l'invention sont employés sous forme de compositions pharmaceutiques qui renferment à titre de principe actif au moins un composé de formule générale I, en association ou en mélange avec un excipient ou un véhicule inerte, non toxique, pharmaceutiquement acceptable.

Les compositions pharmaceutiques selon l'invention sont destinées à l'administration par voie parentérale, digestive, rectale, permuqueuse ou percutanée.

Ils seront donc présentés sous forme de solutés ou de suspensions injectables ou flacons multi-doses, sous forme de comprimés nus ou enrobés, de dragées, de capsules, de gélules, de pilules, de cachets, de poudres, de suppositoires ou de capsules rectales, de solutions ou de suspensions, pour l'usage percutané dans un solvant polaire, ou, pour l'usage permuqueux.

Les excipients qui conviennent sont les dérivés de la cellulose ou la cellulose microcristalline, les carbonates alcalino-terreux, le phosphate de magnesium, les amidons, les amidons modifiés ou le lactose pour les formes solides.

Pour l'usage rectal, le beurre de cacao ou les stéréates de polyéthylène glycol sont les excipients préférés.

Pour l'usage parentéral, l'eau, les solutés aqueux, le sérum physiologique, les solutés isotoniques sont les véhicules les plus commodément utilisés.

La posologie peut varier dans des limites importantes en fonction de l'indication thérapeutique et de la voie d'administration, ainsi que de l'âge et du poids du sujet.

En règle générale, la posologie unitaire pourra s'échelonner de 1 à 200 mg par prise et la posologie journalière pourra s'échelonner de 2 à 500 mg.

Les exemples suivants illustrent l'invention sans toutefois la limiter.

### EXEMPLE I : 5-(2-phénoxy éthyl) thiazolidine-2,4-dione (I) (selon la voie B)

### STADE A

### Préparation du 2-imino-5-(2-phénoxy éthyl)-4-thiazolidinone (IX)

On chauffe au reflux pendant 3 h. 18,5 g de l'ester éthylique de l'acide 2-bromo-4-phénoxybutanoïque. 6 g de thiourée et 5 g d'acétate de sodium dans 100 ml de 2-méthoxyéthanol. On refroidit et concentre le milieu réactionnel sous vide et le résidu obtenu est repris dans 75 ml d'eau déminéralisée et 75 ml de dichlométhane. La phase organique est décantée, lavée et séchée sur sulfate de sodium pour donner une huile. Par cristallisation dans l'acétonitrile cette huile donne 6 g de 2-imino-5-(2-phénoxyéthyl)-4-thiazolidinone sous forme d'un solide qui se décompose au-dessus de 250°C.

### STADE B :

### Préparation du 5-(2-phénoxy éthyl) thiazolidine-2,4-dione (I)

On chauffe au reflux pendant 8 h. 6 g de 2-imino-5-(2-phénoxy éthyl) 4-thiazolidinone dans 100 ml d'acide chlorhydrique 2N. Un solide cristallise. Ce solide est filtré et lavé à l'eau, puis purifié par recristallisation dans un mélange cyclohexane/acétate d'éthyle pour donner 3 g de 5-(2-phénoxyéthyl) thiazolidine-2,4-dione hémihydrate, fondant à 105-107°C.

La 5-(2-phénoxy éthyl) thiazolidine-2,4-dione a également été obtenu par le procédé de synthèse selon la voie C. Ce composé est obtenu anhydre et fond alors à 81-83°C.

### EXEMPLE II : 5-[1-méthyl-2-phénoxyméthyl] thiazolidine-2,4-dione (I) (selon la voie F)

### STADE A :

### Préparation du 5-[1-méthvl-2-phénoxyéthylidène] thiazolidine-2,4-dione (XVII)

29,3 g de thiazolidine-2,4-dione et 34,2 ml de phénoxy-2 propanone sont portés au reflux dans 500 ml de toluène pendant 20 h. en présence de 2,5 ml de pipéridine et 1,3 I d'acide acétique. On ajoute 4,75 g d'acide paratoluène sulfonique monohydrate et on porte à nouveau au reflux pendant 20 h. en éliminant l'eau formée à l'aide d'un appareil de Dean-Stark. On ajoute ensuite 1000 ml d'eau. La phase organique est décantée, lavée deux fois à l'eau, séchée sur sulfate de sodium, et évaporée. Le résidu est purifié sur une colonne de silice en éluant avec un mélange dichlorométhane/acétone (90/10 en volume). Le solide est repris dans de l'oxyde de diisopropyle et donne, après filtration, 17 g de 5-[1-méthyl-2-phénoxyéthylidène] thiazolidine-2,4-dione sous forme d'un solide jaune dont le point de fusion est 153-155°C.

### STADE B :

### Obtention de la -5-[1-méthvl-2-phénoxyéthyl] thiazolidine-2,4-dione (I)

9 g de 5-[1-méthyl-2-phénoxyéthylidène] thiazolidine-2,4-dione sont hydrogénés à 100°C sous 40-50 bars de pression, pendant 100 h. dans le dioxanne, en présence de 9 g de charbon palladié à 10 %.
Le milieu réactionnel est filtré sur Célite et évaporé sous vide pour donner 5,5 de 5-[1-méthyl-2-phénoxyéthyl] thiazolidine-2,4-dione dont le point de fusion est 106-108° C.

### EXEMPLE III : ETUDE PHARMACOLOGIQUE DES COMPOSES DE L'INVENTION

### 1 - BUT DE L'EXPERIMENTATION

Déterminer une activité antidiabétique par voie orale sur un modèle expérimental de diabète non insulinodépendant, induit chez le rat par la Streptozotocine.

### 2 - PROTOCOLE

### Méthode d'obtention du modèle rat n0STZ

Le modèle de diabète non insulinodépendant est obtenu chez le rat par une injection ° néonatale (le jour de la naissance) de Streptozotocine.
Les rats diabétiques utilisés sont âgés de 8 semaines.
La stabulation des animaux est réalisée, du jour de leur naissance au jour de l'expérimentation, dans une animalerie à température régulée de 21 à 22° C, et soumise à un cycle fixe de lumière (de 7H à 19H) et d'obscurité de (19H à 7H). Leur alimentation a consisté en un régime d'entretien ; eau et nourriture ont été fournies « ad libitum », à l'exception du jeûne de 2 heures précédant les tests où la nourriture est retirée.

### METHODE

Le jour de l'expérimentation, les rats sont traités par voie orale avec le produit à tester, puis 90' après, sont anesthésiés au Nembutal. 2 heures après l'administration du produit, un prélèvement sanguin de 500 µl est effectué au niveau de la queue.

### RECUEIL DES ECHANTILLONS

Le sang est recueilli sur Héparine. Tous les tubes sont placés dans la glace au moment du recueil des prélèvements. Ces derniers sont ensuite centrifugés 10 minutes à 3000 tours/minute pour séparer les éléments figurés le plus rapidement possible.
Le plasma obtenu est réparti en 2 aliquotes :
⇒l'un pour le dosage de la glycémie et de la lactatémie ; ces dosages sont réalisés immédiatement
⇒l'autre pour le dosage de l'insulinémie - est stocké au congélateur à -20° C jusqu'au jour du dosage

### PROCEDURE ANALYTIQUE

### - Glucose et lactate

Ils sont déterminés par la technique de la glucose-oxydase et de la lactate oxydase (Eppendorf Ebio 6666)

### - L'insuline

L'insuline plasmatique est dosée par une méthode radioimmunologique

### 3 - RESULTATS

Le tableau I rassemble les principaux résultats obtenus.

**TABLEAU I**

| **COMPOSE** | 20 mg/kg/4 j | | 200 mg/kg/4 j | |
|---|---|---|---|---|
| | % Glycémie | % Insulinémie | % Glycémie | % Insulinémie |
| 1 | -15 | | -26 | |
| 6 | -20 | 0 | -26 | -49 |
| 8 | -3 | -30 | -9 | -26 |
| 11 | -12 | -31 | -21 | -23 |
| 15 | -14 | -9 | -22 | -12 |
| 18 | -8 | -4 | -9 | -17 |
| 20 | -10 | | -14 | |
| 21 | -1 | | -9 | |

Ces résultats montrent l'efficacité des composés selon l'invention pour faire diminuer la glycémie spontanée et le taux d'insuline circulant chez les animaux rendus diabétiques.

### ACTION SUR LES PPARγ

Les dérivés de thiazolidinedione sont des agents antidiabétiques qui augmentent la sensibilité à l'insuline des tissus cibles dans des modèles animaux de diabète non insulinodépendant. Les thiazolidinediones sont connues pour favoriser in vitro la différentiation en adipocytes des lignées cellulaires préadipocytes et mésenchymateuses ; cependant, la base moléculaire de cet effet adipogénique reste incertain. Les thiazolidinediones sont des activateurs puissants et sélectifs du récepteur γ activé par un proliférateur de peroxisome (= PPARγ : « Peroxisome Proliferator - activated Receptor γ »), un membre de la « super » famille des récepteurs nucléaires dont on a récemment démontré l'action dans l'adipogénèse. Le plus puissant de ces agents, la thiazolidinedione BRL 49653, se lie à PPARγ avec une constante de dissociation Kd approximativement égale à 40 nM. Le traitement de la lignée cellulaire C3H10T1/2 par BRL 49653 a pour résultat une différentiation efficace en adipocytes. Ceci démontre la grande affinité du ligand PPAR et prouve que PPARγ est une cible moléculaire pour les effets adipogéniques des thiazolidinediones.

A la différence des autres thiazolidinediones connues (troglitazone, pioglitazone, BRL 49653), les thiazolidinediones de la présente invention n'ont aucune activité sur la transactivation du PPARγ. De même, les thiazolidinediones de la présente invention n'ont aucune activité sur la transactivation des autres récepteurs nucléaires PPARs, PPARα, et PPARδ. Les thiazolidinediones de la présente invention sont peu ou pas adipogéniques au niveau des cellules 3T3-L1, contrairement aux thiazolidinediones de l'art antérieur qui favorisent la différentiation des cellules 3T3-L1 en adipocytes.

Ainsi, les composés de la présente invention présentent des propriétés que les composés de la littérature antérieure ne possèdaient pas.

### EXEMPLE IV

Les composés suivants répondant à la formule générale I, ont été préparés par l'une des méthodes de synthèse A, B, C, D, E, ou F. Leurs constantes sont énumérées dans le tableau II ci-après.

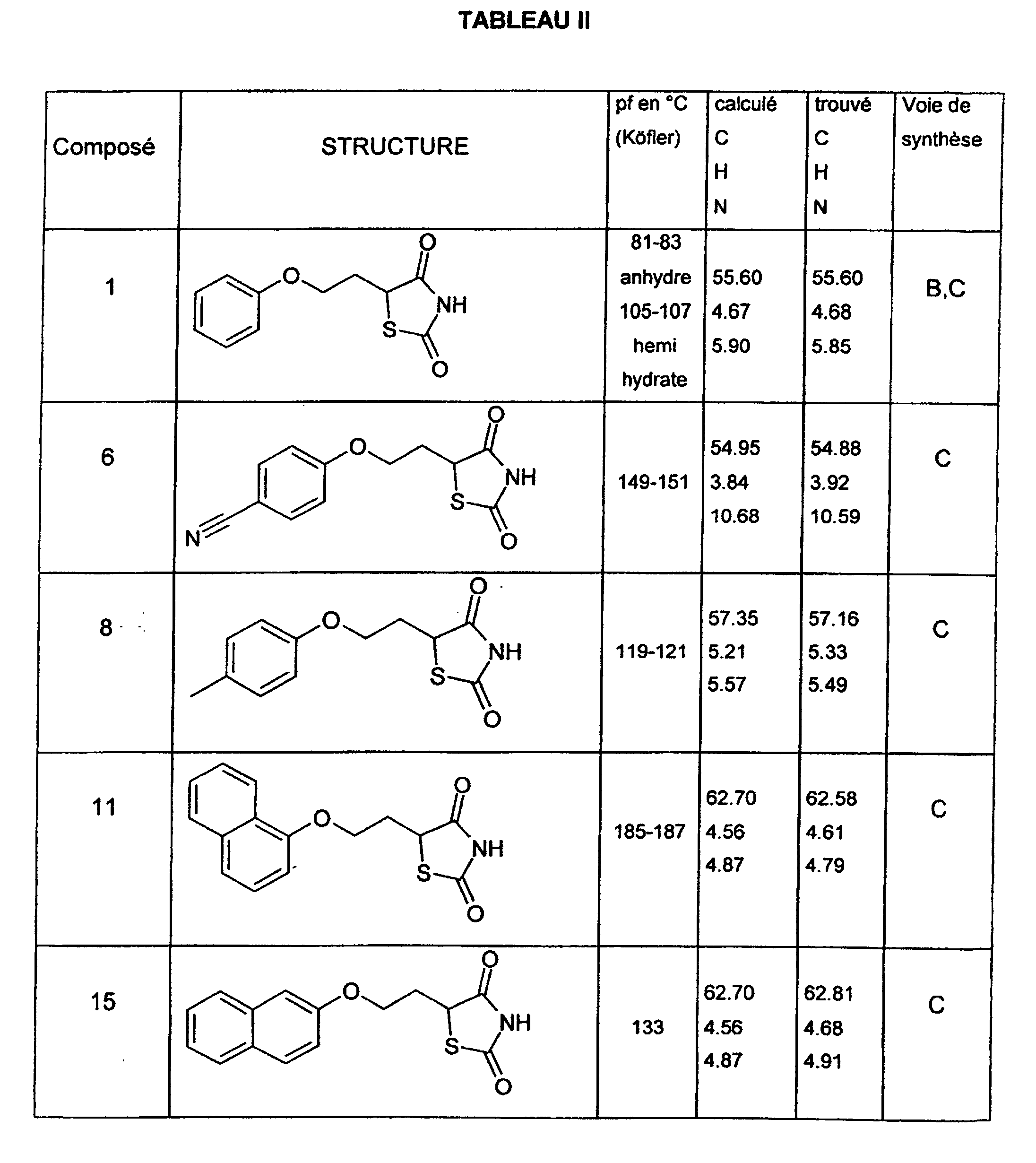

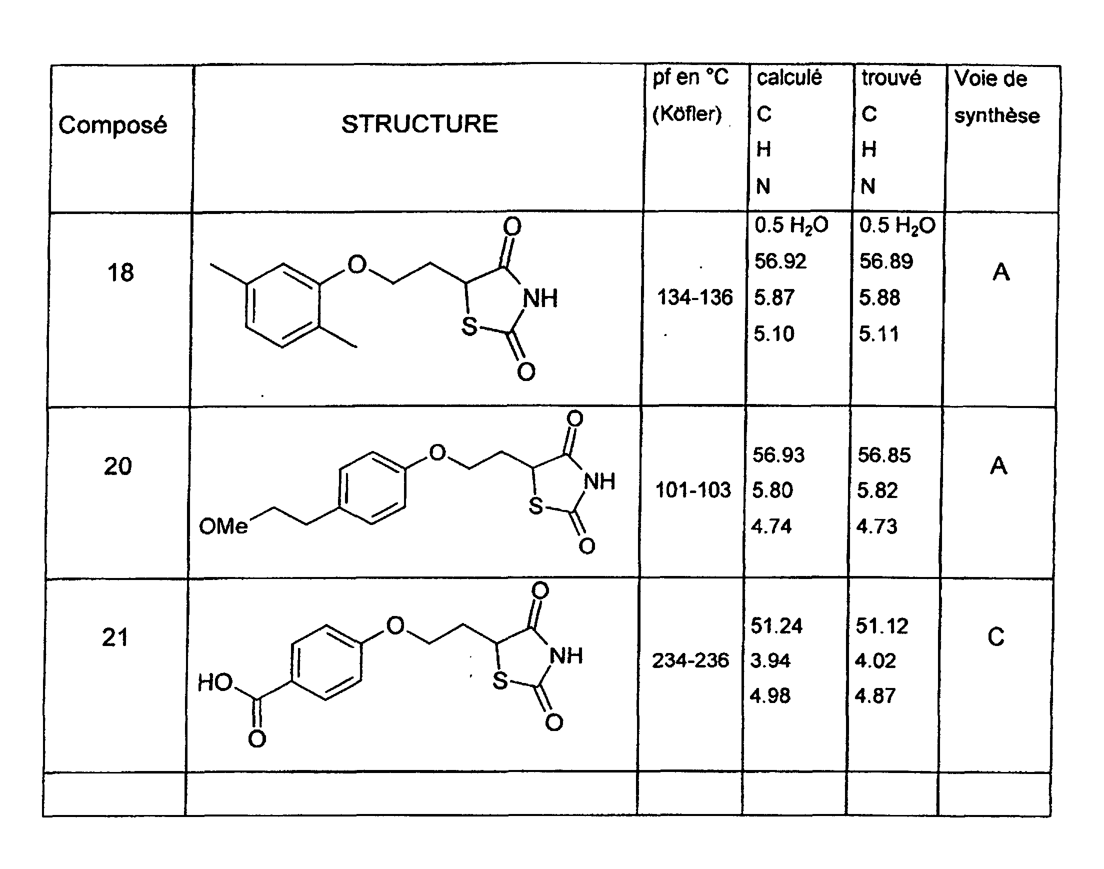

### EXEMPLE V

### Gélules de 5-(2-phénoxy éthyl) thiazolidine-2,4-dione à 50 mg

- Principe actif 50 g
- Lactose 75 g
- Stéarate de magnésium 5 g
pour 1.000 gélules terminées au poids moyen de 0,130 g

### EXEMPLE VI

### Comprimés enrobés de 5-[2-(2-naphtoxy) éthyl] thiazolidine-2,4-dione à 75 mg

- Principe actif 75 g
- Silice 39 g
- Lactose 112 g
- Carboxyméthyl amidon en tant que sel de sodium 9 g
- Talc 8 g
- Stéarate de magnésium 7 g
pour 1.000 noyaux pesant en moyenne 250 g
**Enrobage** :
Gomme laque
Gélatine
Gomme arabique
Saccharose
Dioxyde de titane
Cire d'abeille
Cire de Carnauba
Ethyl vanilline
pour 1.000 comprimés enrobés terminés au poids moyen de 0,400 g.

## Revendications

1. Les nouvelles 5-phénoxyalcoyl-2,4-thiazolidinediones de formule générale I: 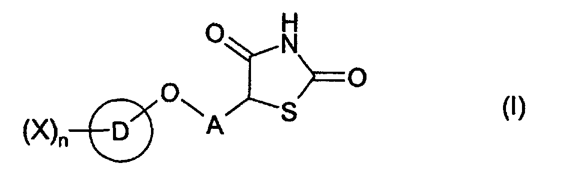 dans laquelle A représente CH₂CH₂, éventuellement substituée par un radical hydroxyle ou par un radical phényle
D représente une structure aromatique mono-, bi- ou tricyclique, homo- ou hétérocarbonée pouvant inclure un ou plusieurs hétéroatomes
X représente un substituant de la structure aromatique choisi parmi l'hydrogène, un groupe alcoyle ayant de 1 à 6 atomes de carbone, un groupe alcoxy ayant de 1 à 6 atomes de carbone, un groupe alcoxyalcoyle dans lequel les groupements alcoxy et alcoyle sont définis comme précédemment, un groupe aryle défini comme une structure cyclique aromatique comportant un ou deux cycles incluant éventuellement un ou deux hétéroatomes dans le cycle, un groupe aralcoyle dans lequel le groupement alcoyle est défini comme précédemment et le groupe aryle est défini comme précédemment, un groupe aralcoylaryle dans lequel les groupements aralcoyle et aryle sont définis comme précédemment, un trifluorométhyle, un cyano, un hydroxy, un nitro, un amino, un carboxyle, un alcoxycarbonyle, un carboxamide, un sulfonyl, un sulfone, un sulfonamide, un sulfamoyle, un alcoylsufonylamino, un acylamino, un trifluorométhoxy
n est un nombre entier allant de 1 à 3, sous forme libre ou salifiée.

2. Les formes tautomères des composés de formule générale I selon la revendication 1.

3. Les énantiomères des composés de formule générale I selon la revendication 1.

4. Les diastéréoisomères des composés de formule générale I selon la revendication 1.

5. Les épimères des composés de formule générale I selon la revendication 1.

6. Les solvates avec de l'eau ou un solvant organique des composés de formule générale I selon la revendication 1.

7. Les sels basiques pharmacologiquement acceptables des composés de formule générale I selon l'une des revendications 1 à 5.

8. Un composé selon l'une des revendications 1 à 7, choisi dans le groupe constitué par:
- la 5-(2-phénoxy éthyl) thiazolidine-2,4-dione
- la 5-[1-méthyl-2 phénoxyéthyl] thiazolidine-2,4-dione
- la 5-[2-(4-cyanophénoxy) éthyl] thiazolidine-2,4-dione
- la 5-[2-(2-naphtoxy) éthyl] thiazolidine-2,4-dione
et leurs sels pharmacologiquement acceptables.

9. Un procédé d'obtention des composés de formule générale I selon la revendication 1 : 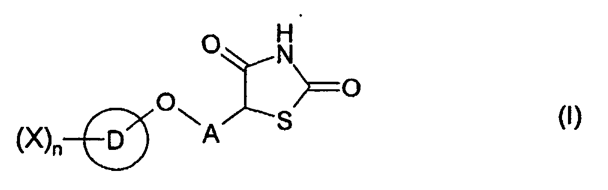 dans laquelle D représente une structure aromatique définie selon la revendication 1,
X représente un substituant de la structure aromatique défini selon la revendication 1,
A représente CH₂CH₂,
n représente un nombre entier allant de 1 à 3,
qui consiste en ce que l'on soumet un composé de formule II : 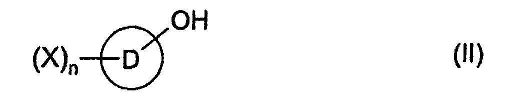 dans laquelle X, D et n sont définis selon la revendication 1,
à l'action d'un dihalogénoalcoyle de formule III : 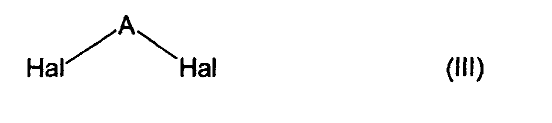 dans laquelle Hal représente un atome de chlore ou de brome,
A représente un radical alcoylène tel que défini précédemment,
en présence d'un agent basique, pour former le composé de formule générale IV : 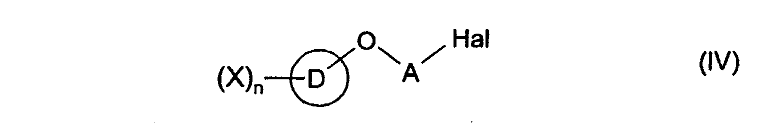 dans laquelle X, D et n sont définis selon la revendication 1, et A est défini comme précédemment,
que l'on soumet à l'action d'un malonate de dialcoyle de formule V : 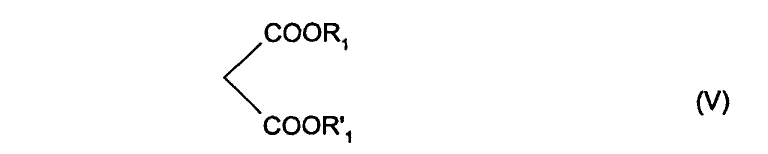 dans laquelle R₁ et R'₁ sont des radicaux alcoyles,
en présence d'un alcoolate de métal alcalin pour former le composé de formule générale VI : 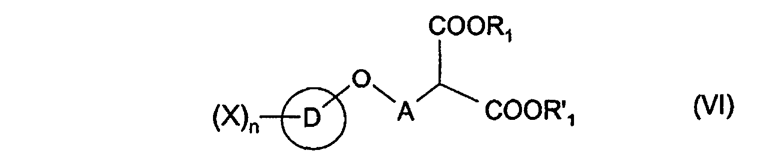 dans laquelle X, D et n sont définis selon la revendication 1,
A, R₁ et R'₁ sont définis comme précédemment,
qui est soumis à une halogénation par un agent d'halogénation pour former le composé de formule générale VII : 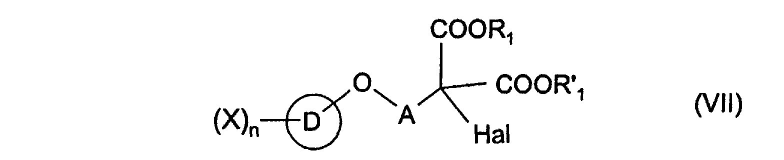 dans laquelle X, D et n sont définis selon la revendication 1,
A, Hal, R₁ et R'₁ sont définis comme précédemment,
qui est chauffé à reflux dans un mélange acide pour obtenir l'α-halogénoacide de formule générale VIII : 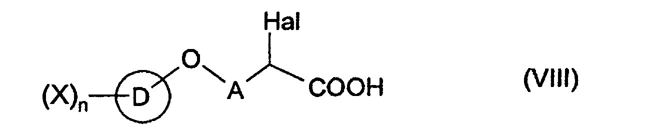 dans laquelle X, D et n et A sont définis selon la revendication 1,
Hal et A sont définis comme précédemment,
qui est mis en réaction avec la thiourée pour obtenir la 2-imino-4-thiazolidinone de formule générale IX : 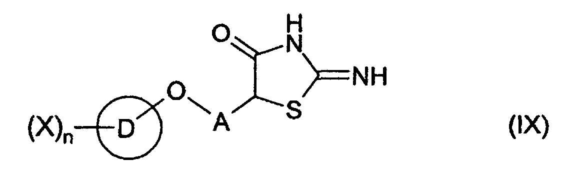 dans laquelle X, D et n et A sont tels que définis selon la revendication 1,
et A est d'fini comme précédemment,
qui est hydrolysée en thiazolidine-2,4-dione de formule générale (I) en milieu acide.

10. Un procédé d'obtention des composés de formule générale (I) selon la revendication 1, dans lequel on soumet un composé de formule X : 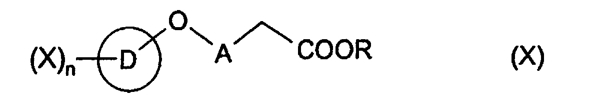 dans laquelle R représente un groupe alcoyle,
X, D, n et A sont définis selon la revendication 1,
à une halogénation pour former l'ester α-halogéné de formule générale XI : 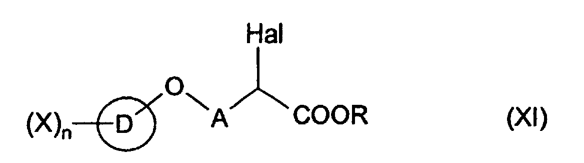 dans laquelle Hal représente un atome de chlore ou de brome,
X, D, n et A sont définis selon la revendication 1, et R représente un group alcoyle,
qui est mis en réaction avec la thiourée pour former la 2-imino-4-thiazolidinone de formule IX : 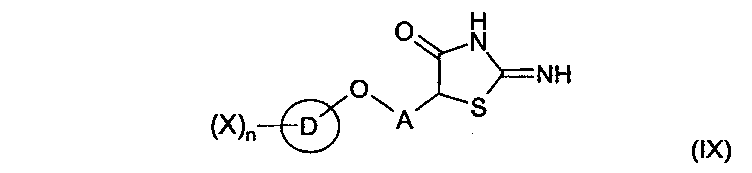 dans laquelle X, D, n et A sont définis selon la revendication 1,
que l'on hydrolyse en un milieu acide pour former le composé de formule générale I.

11. Un procédé d'obtention des composés de formule générale (I) selon la revendication 1, dans lequel on soumet un composé de formule IV tel que défini selon la revendication 9, à l'action du dianion de la thiazolidine-2,4-dione obtenu par action d'un dérivé alcalin sur la thiazolidine-2,4-dione, pour former le composé de formule générale I.

12. Un procédé d'obtention des composés de formule générale (I) selon la revendication 1 dans lequel on soumet le composé aldéhydique de formule générale XII : 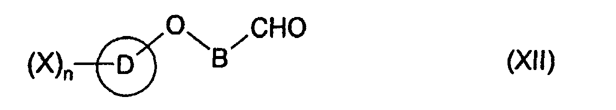 dans laquelle B représente CH₂,
X, D et n sont définis selon la revendication 1,
à l'action du dianion de la thiazolidine-2,4-dione obtenu par action d'un dérivé alcalin sur la thiazolidine-2, 4-dione, pour former le composé de formule générale XIII: 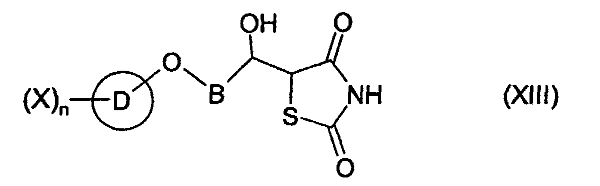 dans laquelle X, D et n et B sont définis selon la revendication 1 et B est défini comme précédemment,
que l'on convertit en dérivé déshydroxylé de formule générale I selon les méthodes connues.

13. Un procédé d'obtention des composés de formule générale (I) selon la revendication 1, dans lequel on soumet une cétone de formule générale XVI : 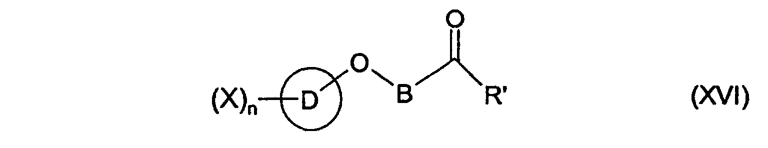 dans laquelle R' représente un groupe alcoyle linéaire ou ramifié, ou un groupe aryle ou aralcoyle, substitués ou non,
X, D et n sont définis selon la revendication 1,
et B est défini selon la revendivation 12,
à l'action de la thiazolidine-2,4-dione en présence d'une base organique puis d'un milieu acide pour former le composé de formule générale XVII : 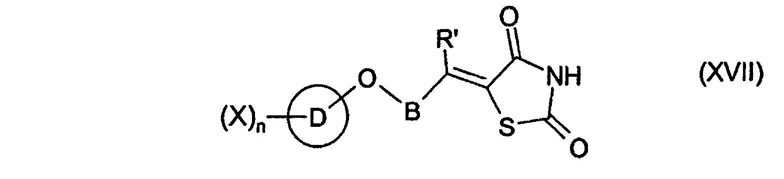 dans laquelle X, D et n sont définis selon la revendication 1,
et B est défini selon la revendication 12,
dans laquelle la double liaison est ensuite hydrogénée par l'action de l'hydrogène en présence d'un catalyseur pour former le composé de formule générale I, dans laquelle la chaîne alcoylène est une chaine ramifiée : 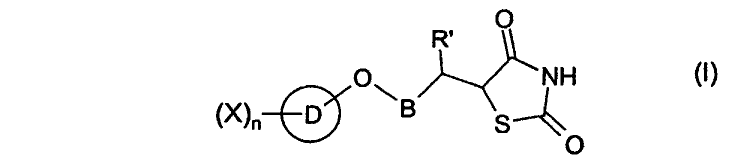 dans laquelle X, D et n sont définis selon la revendication 1,
B est défini selon la revendication 12,
et R' est défini comme précédemment.

14. Les compositions pharmaceutiques **caractérisées en ce qu'**elles renferment à titre de principe actif au moins un composé de formule générale I selon la revendication 1 : 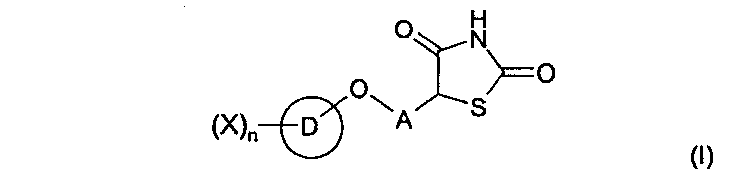 dans laquelle A représente CH₂CH₂, éventuellement substituée par un radical hydroxyle ou par un radical phényle
D représente une structure aromatique mono-, bi- ou tricyclique, homo- ou hétérocarbonée pouvant inclure un ou plusieurs hétéroatomes,
X représente le substituant de la structure aromatique choisi parmi l'hydrogène, un groupe alcoyle ayant de 1 à 6 atomes de carbone, un groupe alcoxy ayant de 1 à 6 atomes de carbone, un groupe alcoxyalcoyle dans lequel les groupements alcoxy et alcoyle sont définis comme précédemment, un groupe aryle défini comme une structure cyclique aromatique comportant un ou deux cycles incluant éventuellement un ou deux hétéroatomes dans le cycle, un groupe aralcoyle dans lequel le groupement alcoyle est défini comme précédemment et le groupement aryle est défini comme précédemment, un groupe aralcoylaryle dans lequel les groupements aralcoyle et aryle sont définis comme précédemment, un trifluorométhyle, un cyano, un hydroxy, un nitro, un amino, un carboxyle, un alcoxycarbonyle, un carboxamide, un sulfonyl, un sulfone, un sulfonamide, un sulfamoyle, un alcoysulfonylamino, un acylamino, un trifluorométhoxy,
n est un nombre entier allant de 1 à 3, sous forme libre ou salifiée.
en association ou en mélange avec un véhicule ou un excipient inerte, non-toxique, pharmaceutiquement acceptable.

15. Une composition pharmaceutique selon la revendication 14 dans laquelle le véhicule ou l'excipient sont un de ceux qui conviennent pour l'administration par voie parentérale, digestive, rectale, permuqueuse ou percutanée.

16. Une composition pharmaceutique selon la revendication 14 ou la revendication 15 dans laquelle la teneur en principe actif varie de 1 à 200 mg par prise unitaire.

## Patentansprüche

1. Neue 5-Phenoxyalkyl-2,4-thiazolidinone der allgemeinen Formel I: 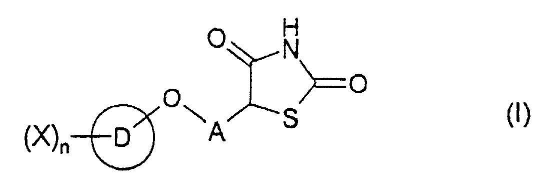 worin A CH₂CH₂, das gegebenenfalls durch einen Hydroxyrest oder einen Phenylrest substituiert ist, bedeutet,
D eine monocyclische, bicyclische oder tricyclische aromatische Homo- oder Heterokohlenstoffstruktur, die ein oder mehrere Heteroatome beinhalten kann, bedeutet,
X einen Substituenten der aromatischen Struktur aus der Gruppe Wasserstoff, Alkylgruppe mit 1 bis 6 Kohlenstoffatomen, Alkoxygruppe mit 1 bis 6 Kohlenstoffatomen, Alkoxyalkylgruppe, in der die Alkoxy- und Alkylgruppierungen wie oben definiert sind, Arylgruppe, die als aromatische cyclische Struktur umfassend einen oder zwei Ringe, gegebenenfalls mit einem oder zwei Heteroatomen in dem Ring, definiert ist, Aralkylgruppe, in der die Alkylgruppierung wie oben definiert ist und die Arylgruppe wie oben definiert ist, Aralkylarylgruppe, in der die Aralkyl- und Arylgruppierungen wie oben definiert sind, Trifluormethyl, Cyan, Hydroxy, Nitro, Amino, Carboxy, Alkoxycarbonyl, Carboxamid, Sulfonyl, Sulfon, Sulfonamid, Sulfamoyl, Alkylsulfonylamino, Acylamino oder Trifluormethoxy bedeutet, und
n eine ganze Zahl von 1 bis 3 bedeutet,
in freier Form oder in Salzform.

2. Tautomeren Formen der Verbindungen der allgemeinen Formel I nach Anspruch 1.

3. Enantiomeren der Verbindungen der allgemeinen Formel I nach Anspruch 1.

4. Diastereoisomeren der Verbindungen der allgemeinen Formel I nach Anspruch 1.

5. Epimeren der Verbindungen der allgemeinen Formel I nach Anspruch 1.

6. Solvate der Verbindungen der allgemeinen Formel I nach Anspruch 1 mit Wasser oder einem organischen Lösungsmittel.

7. Pharmazeutisch unbedenkliche basische Salze der Verbindungen der allgemeinen Formel I nach einem der Ansprüche 1 bis 5.

8. Verbindung nach einem der Ansprüche 1 bis 7 aus der Gruppe
- 5-(2-Phenoxyethyl)thiazolidin-2,4-dion,
- 5-(1-Methyl-2-phenoxyethyl)thiazolidin-2,4-dion,
- 5-[2-(4-Cyanphenoxy)ethyl]thiazolidin-2,4-dion,
- 5-[2-(2-Naphthoxy)ethyl]thiazolidin-2,4-dion
sowie ihre pharmazeutisch unbedenklichen Salze.

9. Verfahren zur Herstellung der Verbindungen der allgemeinen Formel I nach Anspruch 1: 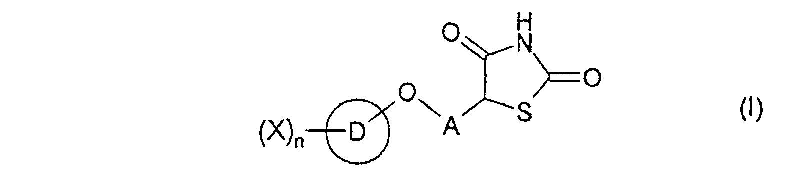 worin D eine wie in Anspruch 1 definierte aromatische Struktur bedeutet,
X einen wie in Anspruch 1 definierten Substituenten der aromatischen Struktur bedeutet,
A CH₂CH₂ bedeutet,
n eine ganze Zahl von 1 bis 3 bedeutet,
das darin besteht, daß man eine Verbindung der Formel II: 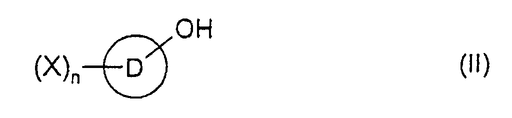 worin X, D und n die in Anspruch 1 angegebenen Bedeutungen haben,
mit einem Dihalogenalkyl der Formel III: 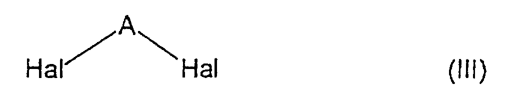 worin Hal ein Chlor- oder Bromatom bedeutet und
A einen wie oben definierten Alkylenrest bedeutet,
in Gegenwart einer Base umsetzt, wodurch man die Verbindung der allgemeinen Formel IV: 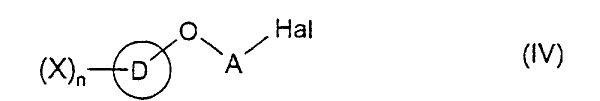 worin X, D und n die in Anspruch 1 angegebenen Bedeutungen haben und A wie oben definiert ist,
erhält,
welche man mit einem Dialkylmalonat der Formel V: 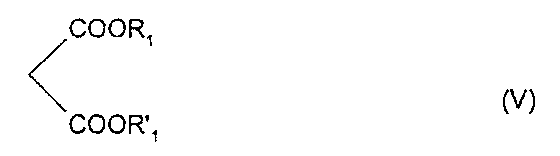 worin R₁ und R'₁ Alkylreste darstellen,
in Gegenwart eines Alkalimetallalkoholats umsetzt, wodurch man die Verbindung der allgemeinen Formel VI: 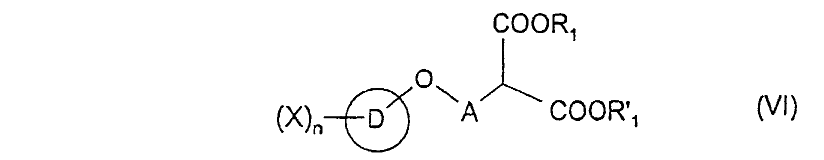 worin X, D und n die in Anspruch 1 angegebenen Bedeutungen haben und
A, R₁ und R'₁ wie oben definiert sind,
erhält,
welche man mit einem Halogenierungsmittel halogeniert, wodurch man die Verbindung der allgemeinen Formel VII: 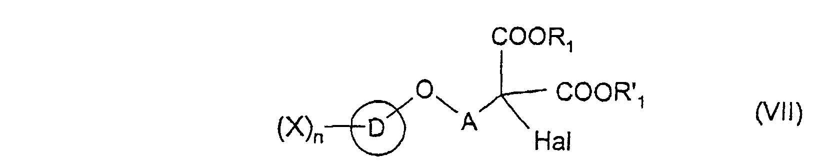 worin X, D und n die in Anspruch 1 angegebenen Bedeutungen haben und
A, Hal, R₁ und R'₁ wie oben definiert sind,
erhält
die man in einer sauren Mischung unter Rückfluß erhitzt, wodurch man die α-Halogensäure der allgemeinen Formel VIII: 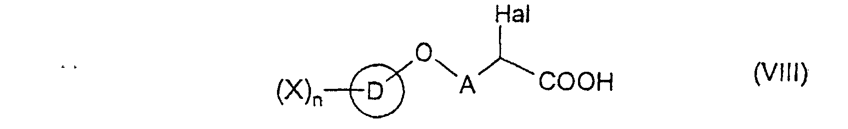 worin X, D und A die in Anspruch 1 angegebenen Bedeutungen haben und
Hal und A wie oben definiert sind,
erhält,
die man mit Thioharnstoff umsetzt, wodurch man das 2-Imino-4-thiazolidinon der allgemeinen Formel IX: 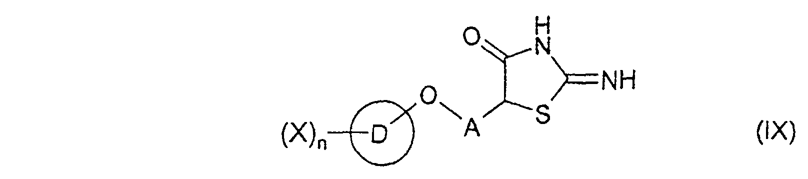 worin X, D, n und A die in Anspruch 1 angegebenen Bedeutungen haben und
A die oben angegebene Bedeutung hat,
erhält,
welches man in einem sauren Medium zu dem Thiazolidin-2,4-dion
der allgemeinen Formel (I) hydrolysiert.

10. Verfahren zur Herstellung der Verbindungen der allgemeinen Formel (I) nach Anspruch 1, bei dem man eine Verbindung der Formel X: 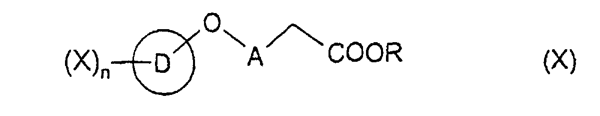 worin R eine Alkylgruppe bedeutet und
X, D, n und A die in Anspruch 1 angegebenen Bedeutungen haben,
halogeniert, wodurch man den α-Halogenester der allgemeinen Formel XI 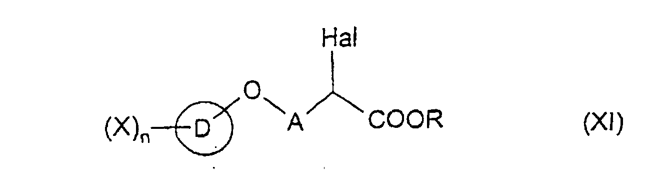 worin Hal ein Chlor- oder Bromatom bedeutet,
X, D, n und A die in Anspruch 1 angegebenen Bedeutungen haben und
R eine Alkylgruppe bedeutet,
erhält,
den man mit dem Thioharnstoff umsetzt, wodurch man das 2-lmino-4-thiazolidinon der Formel IX: 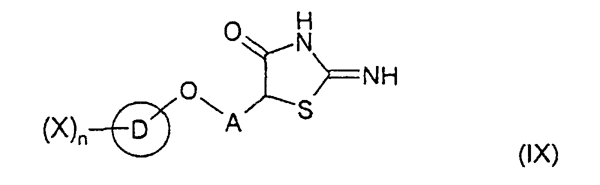 worin X, D, n und A die in Anspruch 1 angegebenen Bedeutungen haben,
erhält,
das man in einem sauren Medium hydrolysiert, wodurch man die Verbindung der allgemeinen Formel I erhält.

11. Verfahren zur Herstellung von Verbindungen der allgemeinen Formel (I) nach Anspruch 1, bei dem man eine Verbindung der Formel IV wie in Anspruch 9 definiert mit dem durch Umsetzen eines Alkaliderivats mit dem Thiazolidin-2,4-dion erhaltenen Dianion des Thiazolidin-2,4-dions umsetzt, wodurch man die Verbindung der allgemeinen Formel I erhält.

12. Verfahren zur Herstellung der Verbindungen der allgemeinen Formel (I) nach Anspruch 1, bei dem man die Aldehydverbindung der allgemeinen Formel XII: 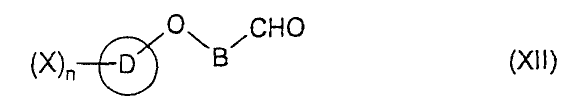 worin B CH₂ bedeutet und
X, D und n die in Anspruch 1 angegebenen Bedeutungen haben,
mit den durch Umsetzen eines Alkaliderivats mit dem Thiazolidin-2,4-dion erhaltenen Dianion des Thiazolidin-2,4-dions umsetzt, wodurch man die Verbindung der allgemeinen Formel XIII: 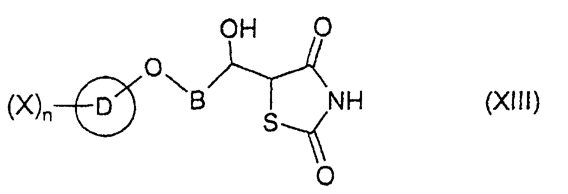 worin X, D und n die in Anspruch 1 angegebenen Bedeutungen haben und B die oben angegebene Bedeutung hat,
erhält,
die man nach bekannten Verfahren in das Deshydroxylderivat der allgemeinen Formel I umwandelt.

13. Verfahren zur Herstellung der Verbindungen der allgemeinen Formel (I) nach Anspruch 1, bei dem man ein Keton der allgemeinen Formel XVI: 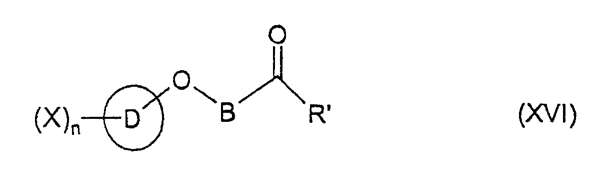 worin R' eine geradkettige oder verzweigte Alkylgruppe oder eine Aryl- oder Aralkylgruppe, die gegebenenfalls substituiert sein können, bedeutet,
X, D und n die in Anspruch 1 angegebenen Bedeutungen haben und B die in Anspruch 12 angegebene Bedeutung hat,
in Gegenwart einer organischen Base und dann eines sauren Mediums mit dem Thiazolidin-2,4-dion umsetzt, wodurch man die Verbindung der allgemeinen Formel XVII: 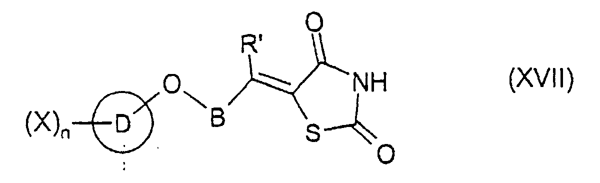 worin X, D und n die in Anspruch 1 angegebenen Bedeutungen haben
und B die in Anspruch 12 angegebene Bedeutung hat,
erhält,
in der die Doppelbindung anschließend mittels Wasserstoff in Gegenwart eines Katalysators hydriert wird, wodurch man die Verbindung der allgemeinen Formel I, worin
die Alkylenkette eine verzweigte Kette: 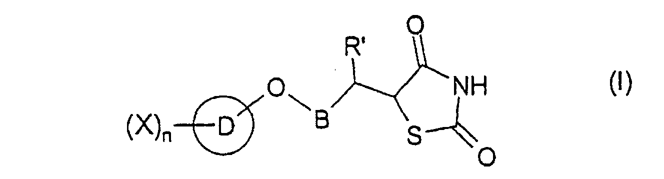 worin X, D und n die in Anspruch 1 angegebenen Bedeutungen haben,
B die in Anspruch 12 angegebene Bedeutung hat
und R' die oben angegebene Bedeutung hat,
bedeutet,
erhält.

14. Pharmazeutische Zusammensetzungen, **dadurch gekennzeichnet, daß** sie als Wirkstoff mindestens eine Verbindung der allgemeinen Formel I nach Anspruch 1: 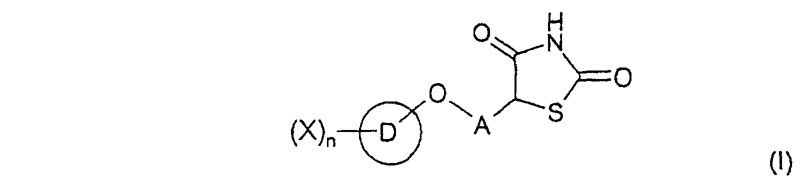 worin A CH₂CH₂, das gegebenenfalls durch einen Hydroxyrest oder einen Phenylrest substituiert ist, bedeutet,
D eine monocyclische, bicyclische oder tricyclische aromatische Homo- oder Heterokohlenstoffstruktur, die ein oder mehrere Heteroatome beinhalten kann, bedeutet,
X einen Substituenten der aromatischen Struktur aus der Gruppe Wasserstoff, Alkylgruppe mit 1 bis 6 Kohlenstoffatomen, Alkoxygruppe mit 1 bis 6 Kohlenstoffatomen, Alkoxyalkylgruppe, in der die Alkoxy- und Alkylgruppierungen wie oben definiert sind, Arylgruppe, die als aromatische cyclische Struktur umfassend einen oder zwei Ringe, gegebenenfalls mit einem oder zwei Heteroatomen in dem Ring, definiert ist, Aralkylgruppe, in der die Alkylgruppierung wie oben definiert ist und die Arylgruppe wie oben definiert ist, Aralkylarylgruppe, in der die Aralkyl- und Arylgruppierungen wie oben definiert sind, Trifluormethyl, Cyan, Hydroxy, Nitro, Amino, Carboxy, Alkoxycarbonyl, Carboxamid, Sulfonyl, Sulfon, Sulfonamid, Sulfamoyl, Alkylsulfonylamino, Acylamino oder Trifluormethoxy bedeutet, und
n eine ganze Zahl von 1 bis 3 bedeutet,
in freier Form oder in Salzform
in Kombination oder als Mischung mit einem inerten pharmazeutisch unbedenklichen Träger- und/oder Hilfsstoff
enthalten.

15. Pharmazeutische Zusammensetzung nach Anspruch 14, bei der sich der Träger- und/oder Hilfsstoff zur parenteralen Verabreichung, Verabreichung über das Verdauungssystem, rektalen Verabreichung, Verabreichung über die Schleimhäute oder zur perkutanen Verabreichung eignet.

16. Pharmazeutische Zusammensetzung nach Anspruch 14 oder Anspruch 15, bei der der Wirkstoffgehalt im Bereich von 1 bis 200 mg pro Einzeldosis liegt.

## Claims

1. Novel 5-phenoxyalkyl-2,4-thiazolidinediones of the general formula I: 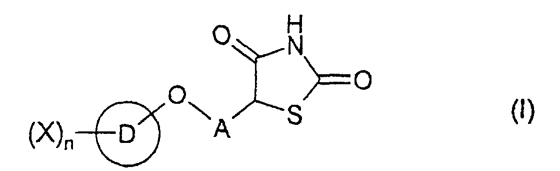 in which A represents CH₂CH₂, optionally substituted with a hydroxyl radical or with a phenyl radical,
D represents a homocarbon or heterocarbon monocyclic, bicyclic or tricyclic aromatic structure possibly including one or more hetero atoms,
X represents a substituent on the aromatic structure chosen from hydrogen, an alkyl group containing from 1 to 6 carbon atoms, an alkoxy group containing from 1 to 6 carbon atoms, an alkoxyalkyl group in which the alkoxy and alkyl groups are defined as above, an aryl group defined as an aromatic cyclic structure comprising one or two rings optionally including one or two hetero atoms in the ring, an aralkyl group in which the alkyl group is defined as above and the aryl group is defined as above, an aralkylaryl group in which the aralkyl and aryl groups are defined as above, a trifluoromethyl, a cyano, a hydroxyl, a nitro, an amino, a carboxyl, an alkoxycarbonyl, a carboxamide, a sulfonyl, a sulfone, a sulfonamide, a sulfamoyl, an alkylsulfonylamino, an acylamino or a trifluoromethoxy,
n is an integer ranging from 1 to 3, in free or salified form.

2. The tautomeric forms of the compounds of the general formula I according to Claim 1.

3. The enantiomers of the compounds of the general formula I according to Claim 1.

4. The diastereoisomers of the compounds of the general formula I according to Claim 1.

5. The epimers of the compounds of the general formula I according to Claim 1.

6. The solvates of the compounds of the general formula I according to Claim 1 with water or an organic solvent.

7. The pharmacologically acceptable basic salts of the compounds of the general formula I according to one of Claims 1 to 5.

8. Compound according to one of Claims 1 to 7, selected from the group consisting of:
- 5-(2-phenoxyethyl)thiazolidine-2,4-dione
- 5-(1-methyl-2-phenoxyethyl)thiazolidine-2,4-dione
- 5-[2-(4-cyanophenoxy)ethyl]thiazolidine-2,4-dione
- 5-[2-(2-naphthoxy)ethyl]thiazolidine-2,4-dione
and the pharmacologically acceptable salts thereof.

9. Process for obtaining the compounds of the general formula I according to Claim 1: 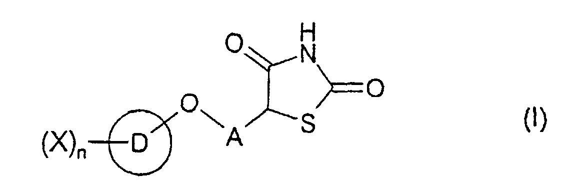 in which D represents an aromatic structure defined according to Claim 1,
X represents a substituent on the aromatic structure defined according to Claim 1,
A represents CH₂CH₂,
n represents an integer ranging from 1 to 3,
which consists in that a compound of the formula II: 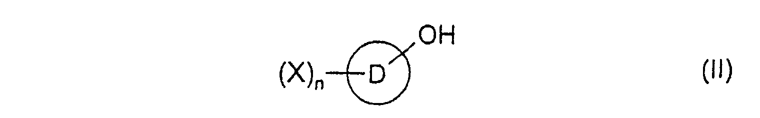 in which X, D and n are as defined in Claim 1,
is subjected to the action of a dihaloalkyl of the formula III: 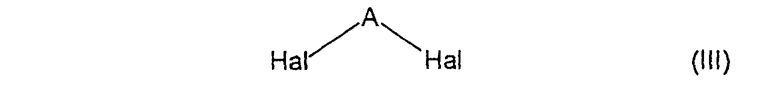 in which Hal represents a chlorine or bromine atom,
A represents an alkylene radical as defined above,
in the presence of a basic agent, to form the compound of the general formula IV: 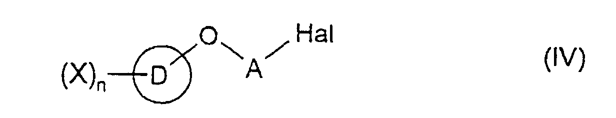 in which X, D and n are as defined in Claim 1, and A is defined as above,
which is subjected to the action of a dialkyl malonate of the formula V: 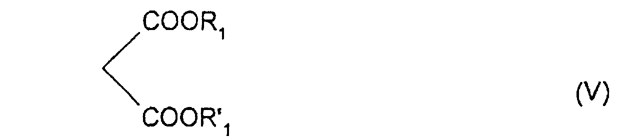 in which R₁ and R'₁ are alkyl radicals,
in the presence of an alkali metal alkoxide to form the compound of the general formula VI: 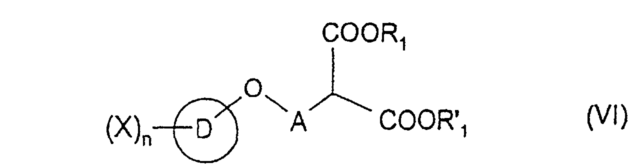 in which X, D and n are as defined in Claim 1,
A, R₁ and R'₁ are defined as above,
which is subjected to a halogenation with a halogenating agent to form the compound of the general formula VII: 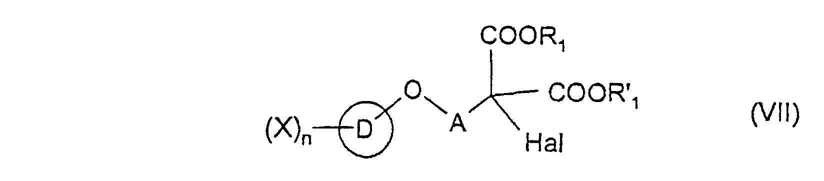 in which X, D and n are as defined in Claim 1,
A, Hal, R₁ and R'₁ are defined as above,
which is refluxed in an acidic mixture to give the α-halo acid of the general formula VIII: 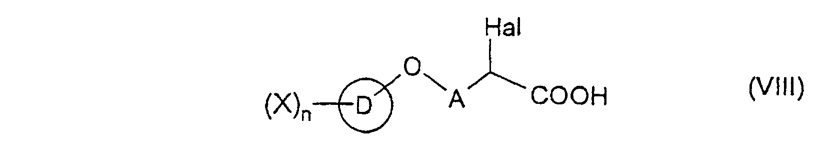 in which X, D, n and A are as defined in Claim 1,
Hal and A are defined as above,
which is reacted with thiourea to give the 2-imino-4-thiazolidinone of the general formula IX: 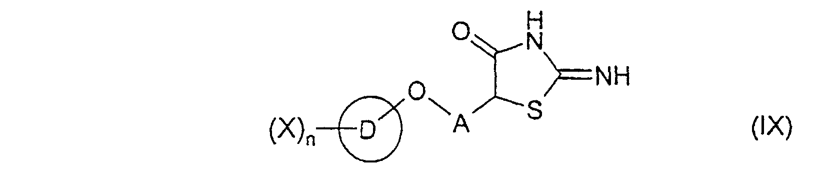 in which X, D, n and A are as defined according to Claim 1,
and A is defined as above,
which is hydrolysed in acidic medium to the thiazolidine-2,4-dione of the general formula (I).

10. Process for obtaining the compounds of the general formula (I) according to Claim 1, in which a compound of the formula X: 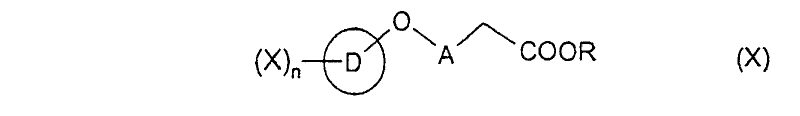 in which R represents an alkyl group,
X, D, n and A are as defined in Claim 1,
is subjected to a halogenation to form the a-halo ester of the general formula XI: 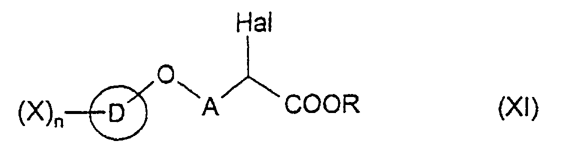 in which Hal represents a chlorine or bromine atom,
X, D, n and A are as defined in Claim 1,
and R represents an alkyl group,
which is reacted with thiourea to form the 2-imino-4-thiazolidinone of the formula IX: 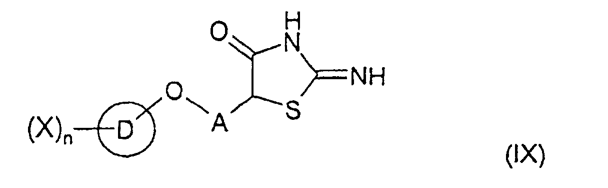 in which X, D, n and A are as defined in Claim 1,
which is hydrolysed in acidic medium to form the compound of the general formula I.

11. Process for obtaining the compounds of the general formula (I) according to Claim 1, in which a compound of the formula IV as defined according to Claim 9 is subjected to the action of the dianion of thiazolidine-2,4-dione obtained by the action of an alkaline derivative on thiazolidine-2,4-dione, to form the compound of the general formula I.

12. Process for obtaining the compounds of the general formula (I) according to Claim 1, in which the aldehyde compound of the general formula XII: 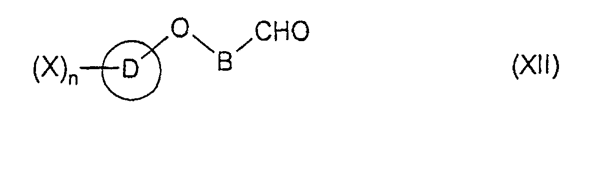 in which B represents CH₂,
X, D and n are as defined in Claim 1,
is subjected to the action of the dianion of thiazolidine-2,4-dione obtained by the action of an alkaline derivative on thiazolidine-2,4-dione, to form the compound of the general formula XIII: 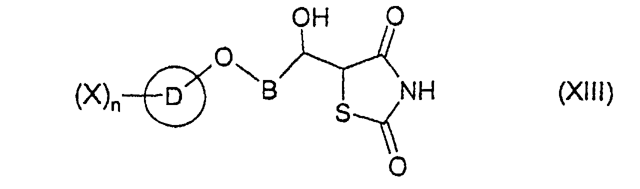 in which X, D, n and B are as defined in Claim 1 and B is defined as above,
which is converted by known methods into the dehydroxylated derivative of the general formula I.

13. Process for obtaining the compounds of the general formula (I) according to Claim 1, in which a ketone of the general formula XVI: 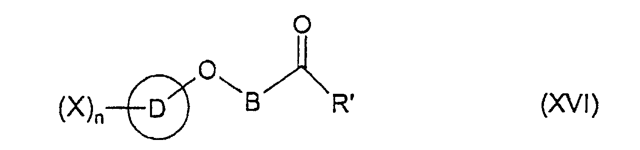 in which R' represents a linear or branched alkyl group or an aryl or aralkyl group, which may be substituted or unsubstituted,
X, D and n are as defined in Claim 1,
and B is as defined in Claim 12,
is subjected to the action of thiazolidine-2,4-dione in the presence of an organic base and then of an acidic medium, to form the compound of the general formula XVII: 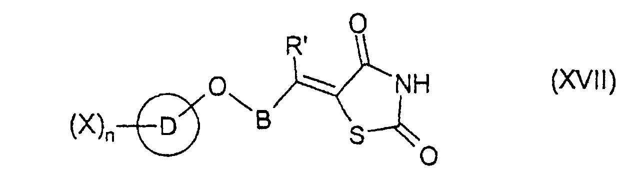 in which X, D and n are as defined in Claim 1,
and B is as defined in Claim 12,
in which the double bond is then hydrogenated by the action of hydrogen in the presence of a catalyst to form the compound of the general formula I, in which the alkylene chain is a branched chain: 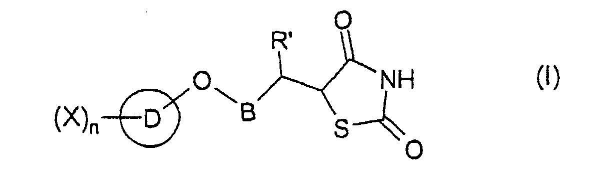 in which X, D and n are as defined in Claim 1,
B is as defined in Claim 12,
and R' is as defined above.

14. Pharmaceutical compositions, **characterised in that** they comprise, as active principle, at least one compound of the general formula I according to Claim 1: 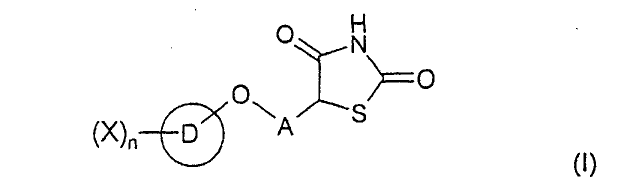 in which A represents CH₂CH₂, optionally substituted with a hydroxyl radical or with a phenyl radical,
D represents a homocarbon or heterocarbon monocyclic, bicyclic or tricyclic aromatic structure possibly including one or more hetero atoms,
X represents a substituent on the aromatic structure chosen from hydrogen, an alkyl group containing from 1 to 6 carbon atoms, an alkoxy group containing from 1 to 6 carbon atoms, an alkoxyalkyl group in which the alkoxy and alkyl groups are defined as above, an aryl group defined as an aromatic cyclic structure comprising one or two rings optionally including one or two hetero atoms in the ring, an aralkyl group in which the alkyl group is defined as above and the aryl group is defined as above, an aralkylaryl group in which the aralkyl and aryl groups are defined as above, a trifluoromethyl, a cyano, a hydroxyl, a nitro, an amino, a carboxyl, an alkoxycarbonyl, a carboxamide, a sulfonyl, a sulfone, a sulfonamide, a sulfamoyl, an alkylsulfonylamino, an acylamino or a trifluoromethoxy,
n is an integer ranging from 1 to 3, in free or salified form,
in combination or as a mixture with an inert, non-toxic, pharmaceutically acceptable vehicle or excipient.

15. Pharmaceutical composition according to Claim 14, in which the vehicle or excipient is one of those that are suitable for parenteral, digestive, rectal, permucous or percutaneous administration.

16. Pharmaceutical composition according to Claim 14 or Claim 15, in which the active principle content ranges from 1 to 200 mg per unit dose.
